# EUROPEAN PATENT SPECIFICATION

(11) **EP 4 088 761 B1**
(45) Date of publication and mention of the grant of the patent: **03.07.2024**
(21) Application number: 22173099.7
(22) Date of filing: 12.05.2022
(51) Int. Cl.: A61M 5/168, A61M 5/20

(54) **RESERVOIR STATE MONITORING**
RESERVOIRZUSTANDSÜBERWACHUNG
SURVEILLANCE D'ÉTAT DE RÉSERVOIR

(30) Priority: 13.05.2021 US 202163187939 P
(43) Date of publication of application: 16.11.2022
(73) Proprietor: LTS Device Technologies Ltd, Netanya 4250529 (IL)
(72) Inventor: BEN-DAVID, Ori, 6311507 Tel-Aviv (IL); PERETZ, Shai, 6299622 Tel-Aviv (IL)
(74) Representative: Thompson, Trevor George

(56) References cited:
- US-A1- 2016 213 834
- US-A1- 2017 368 269
- US-A1- 2018 272 072
- US-A1- 2018 361 082

## Description

### CROSS REFERENCE TO RELATED APPLICATION

The present application claims the priority of US 63/187,939 to Ben-David et al., filed May 13, 2021, entitled, "Reservoir state monitoring."

### FIELD OF THE INVENTION

The present invention relates generally to delivery of a therapeutic substance to a subject, and more specifically to wearable drug delivery devices.

US 2017/0368269A1 discloses a medicinal-liquid administering device for administering medicinal liquid filling a tubular body. The device comprises a disposable part and a reusable part. The disposable part includes a plunger, and a movable part configured to be moved from an initial position and to press the plunger toward a distal-end side. The reusable part is attachable to and detachable from the disposable part. The reusable part includes: a first position detection sensor configured to detect whether or not the movable part is at the initialA position in a state before the movable part is moved; and a second position detection sensor that detects whether or not the movable part moved from the initial position is at a predetermined position at which a remaining amount of the medicinal liquid in the tubular body becomes a predetermined amount.

US 2018/0361082A1 discloses a medicament delivery device having a tubular rotator, a medicament delivery member cover and a sensor arrangement. The tubular rotator includes a guide structure arranged to convert linear motion of the medicament delivery member cover to rotational motion of the tubular rotator, and a profiled distal edge periphery arranged to interact with the sensor arrangement for actuating the sensor arrangement.

US 2018/0272072A1 discloses an electronically controlled add-on device with a switch arrangement comprises a powered switch which is open during most of its lifetime, i.e. during both storage and operational use.

US 2016/0213834A1 discloses an apparatus having a syringe housing. A plunger is slidably received within the syringe housing between a first and second position . A Hall sensor is disposed within a shaft and a magnet is fixed proximate the syringe housing.

### BACKGROUND

Pumps are often used in the medical industry for delivering therapeutic substances, e.g., drugs, to subjects. Therapeutic substances such as saline solution, insulin, antibiotics, and chemotherapy drugs may all be delivered to a subject with medical pumps. While hospitalization is required for delivery of some therapeutic substances, other therapeutic substances, such as for example insulin, do not require that the subject be in the hospital. Wearable medical pumps enable patients to go about their daily lives while receiving a therapeutic substance.

### SUMMARY OF THE INVENTION

A therapeutic substance delivery apparatus, such as a wearable medical patch pump, is provided for delivering a therapeutic substance to a subject. The therapeutic substance delivery apparatus includes a housing, a reservoir disposed at least partially within the housing, and a plunger that is slidable within the reservoir. The therapeutic substance delivery apparatus is typically packaged for commercial sale with the reservoir empty and the apparatus accompanied by a syringe containing a volume of the therapeutic substance. The housing of the therapeutic substance delivery apparatus is typically shaped to define a filling port that is in fluid communication with the reservoir and through which the reservoir is filled with the therapeutic substance from the syringe. In preparation for delivery of the therapeutic substance to the subject, a user, e.g., the subject who will receive the medication, a pharmacist, or a caregiver, fills the reservoir with a volume of therapeutic substance, e.g., a prescribed volume of therapeutic substance.

When the reservoir is empty, the plunger typically resides at a proximal end of the reservoir. As used herein, including in the claims, the proximal end of the reservoir is the end that is closest to the filling port, at which the plunger resides when the reservoir is empty, and to which the plunger returns once the contents of the reservoir have been delivered. As the reservoir is filled via the filling port, the plunger is pushed distally within the reservoir toward a distal end of the reservoir. As used herein, including in the claims, the distal end of the reservoir is the end that is furthest from the filling port, and to which the plunger slides as the reservoir is being filled.

Thus, during operation of the therapeutic substance delivery apparatus, the position of the plunger within the reservoir can provide information regarding the filling of the reservoir and subsequent delivery of the therapeutic substance from the reservoir to the subject. In accordance with some applications of the present invention, in order to monitor the filling of the reservoir and subsequent delivery of the therapeutic substance from the reservoir to the subject, the therapeutic substance delivery apparatus typically has a position sensor having at least three states, and a plunger-sensor engagement interface that is coupled to the plunger within the reservoir and extends from within the reservoir to outside of the reservoir in order to engage the position sensor. The plunger and the position sensor are arranged such that the plunger-sensor engagement interface causes the position sensor to change states as the plunger slides within the reservoir. Control circuitry of the therapeutic substance delivery apparatus identifies new state indications of the apparatus, including for some applications, errors associated with filling and/or delivery, based on (a) a change in state of the position sensor, or (b) a previous or current state of the apparatus in combination with a threshold amount of time having elapsed without the position sensor changing state.

According to the invention, there is provided a therapeutic substance delivery apparatus including:
a housing;
a reservoir disposed at least partially within the housing and configured to hold a therapeutic substance;
a position sensor having three discrete states;
a plunger, slidable within the reservoir;
a plunger-sensor engagement interface that is coupled to the plunger within the reservoir and extends from within the reservoir to outside of the reservoir, the plunger and the position sensor being arranged such that the plunger-sensor engagement interface causes the position sensor to change states as the plunger slides within the reservoir; and
control circuitry configured to identify a new state indication of the apparatus based on (a) a change in state of the position sensor, or (b) a previous or current state of the apparatus in combination with a threshold amount of time having elapsed without the position sensor changing state,
wherein:
   the plunger and the position sensor are arranged such that distal movement of the plunger from an initial proximal position within the reservoir to an intermediate position within the reservoir causes the plunger-sensor engagement interface to change the state of the position sensor from a first state S₁ of the discrete states in which the plunger-sensor engagement interface is not engaged with the position sensor to a second state S₂ of the discrete states in which the plunger-sensor engagement interface is engaged with the position sensor as the plunger slides distally within the reservoir,
   the plunger and the position sensor are arranged such that distal movement of the plunger past the intermediate position to a post-intermediate position within the reservoir causes the plunger-sensor engagement interface to change the state of the position sensor from S₂ back to S₁,
   the plunger and the position sensor are arranged such that, subsequently to the position sensor changing state from S₂ back to S₁, proximal movement of the plunger within the reservoir causes the plunger-sensor engagement interface to change the state of the position sensor from S₁ to a third state S₃ of the discrete states in which the plunger-sensor engagement interface is engaged with the position sensor as the plunger slides proximally within the reservoir, and
   the plunger and the position sensor are arranged such that, subsequently to the position sensor changing from S₁ to S₃, further proximal movement of the plunger causes the plunger-sensor engagement interface to change the state of the position sensor from S₃ back to S₁.

For some applications, the position sensor has exactly three discrete states.

For some applications, the plunger and the position sensor are arranged such that distal movement of the plunger from the initial proximal position less than 0.5 mm toward the intermediate position does not cause the plunger-sensor engagement interface to change the state of the position sensor from the first state to the second state.

For some applications, the post-intermediate position is at least 0.1 mm distal to the intermediate position.

For some applications, the apparatus is in a sleep state when packaged for commercial sale, and the position sensor is in a first state S₁ when the apparatus is in the sleep state.

For some applications, the control circuitry is configured to (a) identify that the apparatus is in an expired state if a sleep-threshold amount of time has elapsed without the position sensor changing state from S₁, and (b) in response thereto, disable the apparatus.

For some applications, the housing is shaped to define a filling port in fluid communication with the reservoir, and the reservoir is configured to be filled with the therapeutic substance via the filling port.

For some applications:
(a) prior to the filling of the reservoir the apparatus is in a sleep state, the plunger is disposed at an initial proximal position within the reservoir, and the position sensor is in state S1,
(b) filling the reservoir with the therapeutic substance causes the plunger to slide in a distal direction from the initial proximal position,
(c) the plunger and the position sensor are arranged such that as the plunger slides in the distal direction, the plunger-sensor engagement interface causes the position sensor to change state from S₁ to S₂, and
(d) the control circuitry is configured to identify that the apparatus is awake and in a filling state in response to the position sensor changing state from S₁ to S₂.

For some applications, the plunger and the position sensor are arranged such that as the plunger slides distally from the initial proximal position, an initial volume of the therapeutic substance enters the reservoir prior to the plunger-sensor engagement interface causing the position sensor to change state from S₁ to S₂.

For some applications:
the plunger and the position sensor are arranged such that upon completion of the reservoir being filled with a threshold volume of the therapeutic substance, the plunger-sensor engagement interface causes the position sensor to change state from S₂ back to S₁, and
the control circuitry is configured to identify that a filling error has occurred in response to (i) the state of the apparatus being filling, in combination with (ii) a filling-threshold amount of time having elapsed from when the position sensor changes state from S₁ to S₂ without the position sensor changing state from S₂ back to S₁.

For some applications, the plunger and the position sensor are arranged such that upon completion of the reservoir being filled with at least a threshold volume of the therapeutic substance, the plunger-sensor engagement interface causes the position sensor to change state from S₂ back to S₁, and the control circuitry is configured to identify that the apparatus is in a filling-complete state in response to the position sensor changing state from S₂ back to S₁.

For some applications, the plunger and the position sensor are arranged such that, subsequently to the plunger-sensor engagement interface causing the position sensor to change state from S₁ to S₂, the plunger-sensor engagement interface maintains engagement with the position sensor, so as to maintain the position sensor in state S₂, until the reservoir has been filled with the threshold volume of the therapeutic substance.

For some applications, the plunger and the position sensor are arranged such that further distal movement of the plunger subsequently to the reservoir being filled with the threshold volume of the therapeutic substance causes the plunger-sensor engagement interface to terminate engagement with the position sensor, causing the position sensor to switch from S₂ back to S₁.

For some applications, a distance between two fixed points of the plunger-sensor engagement interface determines when the plunger-sensor engagement interface terminates engagement with the position sensor.

For some applications:
(a) upon completion of the reservoir being filled with at least a threshold volume of the therapeutic substance, the apparatus is in a filling-complete state, and the position sensor is in state S₁,
(b) subsequently to the apparatus being in the filling-complete state, the control circuitry is configured to initiate a delivery-started state of the apparatus in which the control circuitry drives the therapeutic substance delivery apparatus to deliver the therapeutic substance from the reservoir to a subject by driving the plunger to slide proximally within the reservoir, and
(c) the plunger and the position sensor are arranged such that as the plunger slides proximally the plunger-sensor engagement interface causes the position sensor to change state from S₁ to S₃.

For some applications, the control circuitry is configured to identify that there is a leak in the reservoir in response to the position sensor changing state from S₁ to S₃ prior to the control circuitry initiating the delivery-started state of the apparatus, and in response to the identification that there is a leak, disable the apparatus.

For some applications, the control circuitry is further configured to (a) receive an input indicating an intended volume of the therapeutic substance to be filled within the reservoir, and (b) in response to the received input, determine the length of an expected wait-time between the control circuitry initiating the delivery-started state of the apparatus and the plunger-sensor engagement interface causing the position sensor to change state from S₁ to S₃.

For some applications, the control circuitry is configured to:
in response to (i) the current state of the apparatus being delivery-started, in combination with (ii) the position sensor changing state from S₁ to S₃ prior to the expected wait-time having elapsed, terminate the delivery of the therapeutic substance to the subject.

For some applications, the control circuitry is configured to identify that the reservoir has been filled with a volume of therapeutic substance that is larger than the intended volume in response to (i) the current state of the apparatus being delivery-started, in combination with (ii) the position sensor changing state from S₁ to S₃ after an amount of time that is longer than the expected wait-time but shorter than a delivery-start-threshold amount of time.

For some applications, the control circuitry is configured to terminate the delivery of the therapeutic substance to the subject in response to the reservoir having been filled with the volume of therapeutic substance larger than the intended volume.

For some applications, the control circuitry is configured to compensate for the difference in volume between the intended volume and the volume of therapeutic substance within the reservoir that is larger than the intended volume by terminating the delivery of the therapeutic substance to the subject upon delivering the intended volume to the subject.

For some applications:
(a) the plunger and the position sensor are arranged such that, in response to the reservoir being filled with the intended volume, upon delivering the intended volume to the subject the plunger-sensor engagement interface causes the position sensor to change state from S₃ back to S₁, and
(b) in response to the identification that the reservoir has been filled with a volume of therapeutic substance that is larger than the intended volume, upon delivering the intended volume to the subject, the control circuitry is configured to terminate the delivery of the therapeutic substance to the subject notwithstanding the position sensor remaining in state S₃.

For some applications, the control circuitry is configured to:
identify that a delivery-start error has occurred in response to (i) the current state of the apparatus being delivery-started, in combination with (ii) a delivery-start-threshold amount of time having elapsed from when the control circuitry initiated the delivery-started state without the position sensor changing state from S₁ to S₃, and
in response thereto, disable the apparatus.

For some applications, the control circuitry is configured to identify that the apparatus is in a delivery-in-progress state, in which therapeutic substance is being delivered from the reservoir to the subject, in response to the position sensor changing state from S₁ to S₃ subsequently to the control circuitry initiating the delivery-started state of the apparatus and the expected wait-time having elapsed.

For some applications, the plunger and the position sensor are arranged such that upon completion of delivery of the intended volume of therapeutic substance from the reservoir, the plunger-sensor engagement interface causes the position sensor to change state from S₃ back to S₁, and the control circuitry is configured to identify that the apparatus is in a delivery-complete state in response to the position sensor changing state from S₃ to S₁.

For some applications, the control circuitry is configured to identify that a delivery-progress error has occurred in response to (i) the current state of the apparatus being delivery-in-progress, in combination with (ii) a delivery-progress-threshold amount of time having elapsed from when the position sensor changes state from S₁ to S₃ without the position sensor changing state from S₃ back to S₁.

For some applications, the apparatus is configured such that when the reservoir is in a filled state in which a volume of therapeutic substance is disposed within the reservoir and the position sensor is in state S₁:
(a) the control circuitry is configured to initiate a delivery-started state of the apparatus in which the control circuitry drives the therapeutic substance delivery apparatus to deliver the therapeutic substance from the reservoir to a subject by driving the plunger to slide proximally within the reservoir, and
(b) the plunger and the position sensor are arranged such that as the plunger slides proximally, the plunger-sensor engagement interface causes the position sensor to change state from S₁ to a post-first state.

For some applications, the control circuitry is configured to identify that there is a leak in the reservoir in response to the position sensor changing state from S₁ to the post-first state prior to the control circuitry initiating the delivery-started state of the apparatus.

For some applications, the control circuitry is further configured to (a) receive an input indicating an intended volume of the therapeutic substance, and (b) in response to the received input determine the length of an expected wait-time between the control circuitry initiating the delivery-started state of the apparatus and the plunger-sensor engagement interface causing the position sensor to change state from S₁ to the post-first state.

For some applications, the control circuitry is configured to terminate the delivery of the therapeutic substance to the subject in response to (i) the current state of the apparatus being delivery-started, in combination with (ii) the position sensor changing state from S₁ to the post-first state prior to the expected wait-time having elapsed.

For some applications, the control circuitry is configured to identify that the volume of therapeutic substance within the reservoir was larger than the intended volume in response to (i) the current state of the apparatus being delivery-started, in combination with (ii) the position sensor changing state from S₁ to S₃ after an amount of time that is longer than the expected wait-time but shorter than a delivery-start-threshold amount of time.

For some applications, the control circuitry is configured to terminate the delivery of the therapeutic substance to the subject in response to the reservoir having been filled with the volume of therapeutic substance larger than the intended volume.

For some applications, the control circuitry is configured to compensate for the difference in volume between the intended volume and the volume of therapeutic substance within the reservoir that was larger than the intended volume by terminating the delivery of the therapeutic substance to the subject upon delivering the intended volume to the subject.

For some applications:
(a) the plunger and the position sensor are arranged such that, in response to the reservoir being filled with the intended volume, upon delivering the intended volume to the subject the plunger-sensor engagement interface causes the position sensor to change state from S₃ back to S₁, and
(b) in response to the identification that the reservoir has been filled with a volume of therapeutic substance that is larger than the intended volume, upon delivering the intended volume to the subject, the control circuitry is configured to terminate the delivery of the therapeutic substance to the subject notwithstanding the position sensor remaining in state S₃.

For some applications:
the control circuitry is configured to identify that a delivery-start error has occurred in response to (i) the current state of the apparatus being delivery-started, in combination with (ii) a delivery-start-threshold amount of time having elapsed from when the control circuitry initiated the delivery-started state without the position sensor changing state from S₁ to the post-first state, and
in response thereto, terminate the delivery of the therapeutic substance to the subject.

For some applications, the control circuitry is configured to identify that the apparatus is in a delivery-in-progress state, in which therapeutic substance is being delivered from the reservoir to the subject, in response to the position sensor changing state from S₁ to the post-first state subsequently to the control circuitry initiating the delivery-started state of the apparatus and the expected wait-time having elapsed.

For some applications, the plunger and the position sensor are arranged such that upon completion of delivery of the intended volume of therapeutic substance from the reservoir, the plunger-sensor engagement interface causes the position sensor to change state from the post-first state back to S₁, and the control circuitry is configured to identify that the apparatus is in a delivery-complete state in response to the position sensor changing state from the post-first state back to S₁.

For some applications, the control circuitry is configured to identify that a delivery-progress error has occurred in response to (i) the current state of the apparatus being delivery-in-progress, in combination with (ii) a delivery-progress-threshold amount of time having elapsed from when the position sensor changes state from S₁ to the post-first state without the position sensor changing state from the post-first state back to S₁.

The present invention will be more fully understood from the following detailed description of applications thereof, taken together with the drawings, in which:

### BRIEF DESCRIPTION OF THE DRAWINGS

Fig. 1A is a series of schematic illustrations depicting the filling process of the reservoir, in accordance with some applications of the present invention;
Fig. 1B is a series of schematic illustrations depicting the delivery process of the therapeutic substance from the reservoir to a subject, in accordance with some applications of the present invention;
Fig. 2 is a series of schematic illustrations depicting various examples of scenarios that may occur during filling and subsequent delivery, in accordance with some applications of the present invention; and
Fig. 3 is a state table depicting various states of the therapeutic substance delivery apparatus, in accordance with some applications of the present invention.

### DETAILED DESCRIPTION

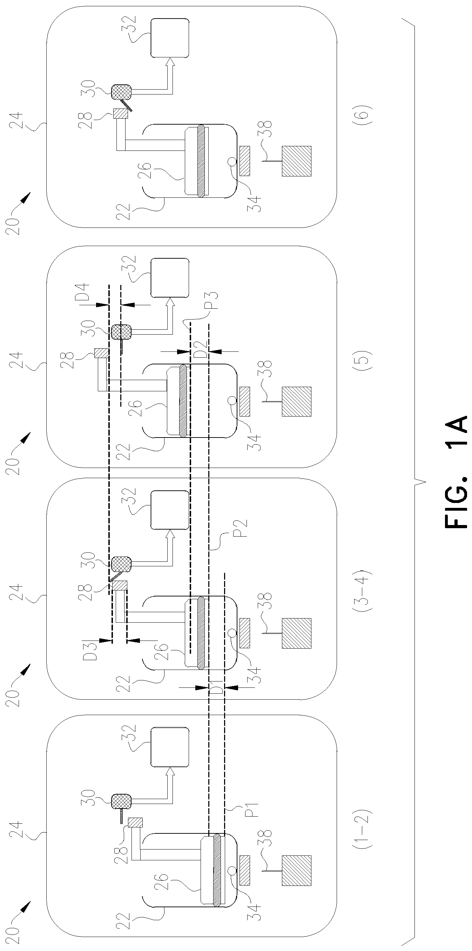

Reference is now made to Figs. 1A-B, which are schematic illustrations that depict, respectively, the filling process of a reservoir 22 of a therapeutic substance delivery apparatus 20, and the delivery process of therapeutic substance from reservoir 22 to a subject (not shown), in accordance with some applications of the present invention. Therapeutic substance delivery apparatus 20 has a housing 24, within which reservoir 22, which is configured to hold a therapeutic substance, is at least partially disposed. A plunger 26 is slidable within reservoir 22.

A plunger-sensor engagement interface 28, e.g., a rigid protrusion, is coupled to plunger 26 within reservoir 22 and extends from within reservoir 22 to outside of reservoir 22 so as to engage with a position sensor 30, e.g., a microswitch, an optical sensor, a magnetic sensor, or an inductive sensor. It is noted that in the case of a magnetic or inductive sensor, plunger-sensor engagement interface 28 includes a magnet or metal portion. Position sensor 30 has at least three states, further described hereinbelow, e.g., at least three discrete states, e.g., exactly three discrete states. Plunger 26 and position sensor 30 are arranged such that plunger-sensor engagement interface 28 causes position sensor 30 to change states as plunger 26 slides within reservoir 22, further described hereinbelow.

As described hereinbelow, control circuitry 32 monitors the state of therapeutic substance delivery apparatus 20 and receives input signals from position sensor 30 corresponding to which state position sensor 30 is in and, for some applications, an amount of time that has elapsed since position sensor 30 last changed state. In response thereto, control circuitry 32 is configured to identify a new state indication of therapeutic substance delivery apparatus 20 based on (a) a change in state of position sensor 30, or (b) a previous or current state of therapeutic substance delivery apparatus 20 in combination with a threshold amount of time having elapsed without position sensor 30 changing state.

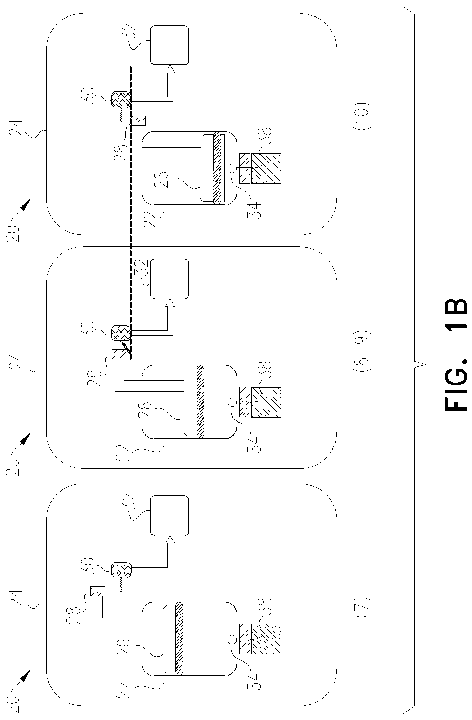

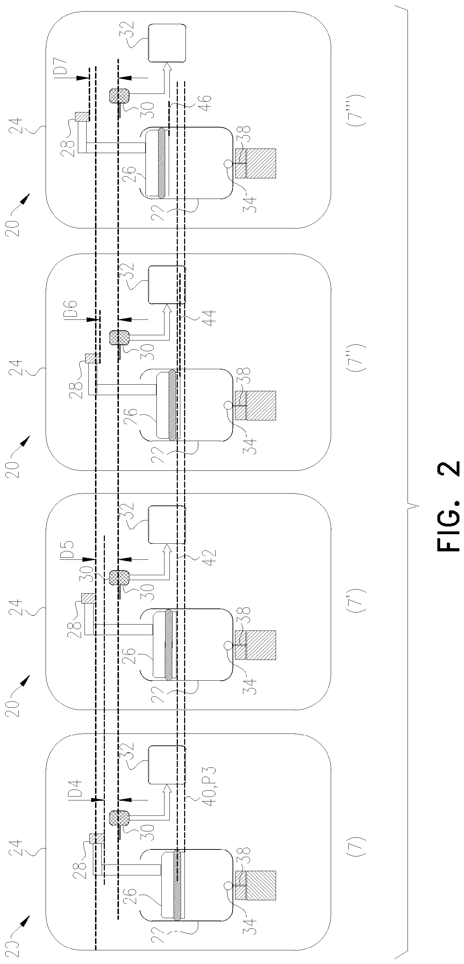

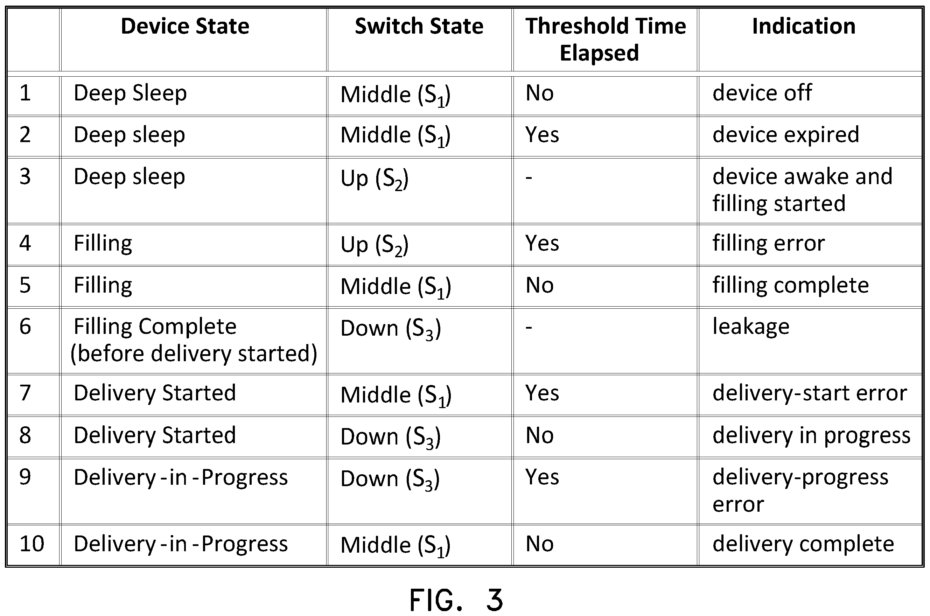

It is noted that throughout the present application, reference is continuously made to the numbered lines of the state table in Fig. 3. The numbers that appear in parentheses under each segment of Figs. 1A, 1B, and 2 correspond to the numbered lines of the state table of Fig. 3. For example, segment (1-2) corresponds to lines 1 and 2 of the state table, and segment (5) corresponds to line 5 of the state table.

It is noted that the terms "first state," and "S₁" are used interchangeably throughout the present application, including in the claims, and refer to a neutral state of position sensor 30 in which plunger-sensor engagement interface 28 is not in engagement with position sensor 30. This is depicted by the horizontal line extending from position sensor 30 in segments (1-2) and (5) of Fig. 1A, and segments (7) and (10) of Fig. 1B.

It is noted that the terms "second state," and "S₂" are used interchangeably throughout the present application, including in the claims, and refer to a state of position sensor 30 in which plunger-sensor engagement interface 28 is engaged with position sensor 30 as plunger 26 slides distally within reservoir 22. This is depicted by the upwards-slanted line (to the upper left) extending from position sensor 30 in segment (3-4) of Fig. 1A.

It is noted that the terms "third state," "S₃," and "post-first state" are used interchangeably throughout the present application, including in the claims, and refer to a state of position sensor 30 in which plunger-sensor engagement interface 28 is engaged with position sensor 30 as plunger 26 slides proximally within reservoir 22. This is depicted by the downwards-slanted line (to the lower left) extending from position sensor 30 in segment (6) of Fig. 1A, and segment (8-9) of Fig. 1B.

Reference is now made specifically to segments (1-2) and (3-4) of Fig. 1A, and to lines 1-3 of the state table of Fig. 3. Prior to the filling of reservoir 22, i.e., when reservoir 22 is empty, plunger 26 typically resides at an initial proximal position P1 within reservoir 22, and position sensor 30 is in state S₁. This is illustrated by the dashed line labeled P1 spanning segments (1-2) and (3-4) of Fig. 1A. Plunger 26 and position sensor 30 are arranged such that distal movement of plunger 26 from initial proximal position P1 within reservoir 22 to an intermediate position P2 within reservoir 22 causes plunger-sensor engagement interface 28 to change the state of position sensor 30 from S₁ to S₂. Intermediate position P2 is illustrated by the dashed line labeled P2 spanning segments (1-2), (3-4), and (5) of Fig. 1A. Typically, distal movement of plunger 26 is caused by reservoir 22 being filled with therapeutic substance via a filling port 34 in housing 24 that is in fluid communication with reservoir 22, i.e., as therapeutic substance flows into reservoir 22 via filling port 34, plunger 26 is pushed distally within reservoir 22.

Therapeutic substance delivery apparatus 20 is typically in a sleep state, with reservoir 22 empty, when packaged for commercial sale. In the sleep state, position sensor 30 is in state S₁. This corresponds to line 1 of the state table of Fig. 3 and is depicted by segment (1-2) of Fig. 1A. Therapeutic substance delivery apparatus 20 is configured such that when plunger 26 slides distally in response to reservoir 22 being filled, the switch of position sensor 30 from S₁ to S₂, caused by plunger-sensor engagement interface 28 engaging position sensor 30, causes therapeutic substance delivery apparatus 20 to wake up from the sleep state. Control circuitry 32 is configured to identify that therapeutic substance delivery apparatus 20 is awake and in a filling state in response to position sensor 30 changing state from S₁ to S₂. This corresponds to line 3 of the state table of Fig. 3, and is depicted by segment (3-4) of Fig. 1A. Therapeutic substance delivery apparatus 20 is typically configured to be a single-use device, and as such the initial filling of reservoir 22 is the only time during operation of therapeutic substance delivery apparatus 20 that position sensor 30 will change state from S₁ to S₂.

For some applications, control circuitry 32 is configured to identify that therapeutic substance delivery apparatus 20 has expired if a sleep-threshold amount of time has elapsed without position sensor 30 changing state from S₁. This corresponds to line 2 of the state table of Fig. 3, and is depicted by segment (1-2) of Fig. 1A, i.e., therapeutic substance delivery apparatus 20 remaining in the state depicted in segment (1-2) of Fig. 1A for longer than the sleep-threshold amount of time. Typically, in response to determining that therapeutic substance delivery apparatus 20 is in an expired state, control circuitry 32 will disable therapeutic substance delivery apparatus 20 and, for some applications, provide an alert that therapeutic substance delivery apparatus 20 has expired.

For some applications, the sleep-threshold amount of time is programmed into therapeutic substance delivery apparatus 20 during manufacturing and relates to a shelf-life of therapeutic substance delivery apparatus 20 itself, regardless of an expiration date of the particular therapeutic substance being used with therapeutic substance delivery apparatus 20. For some applications, the shelf-life of therapeutic substance delivery apparatus 20 may be longer than the expiration date of the particular therapeutic substance. For some applications, control circuitry 32 is configured to receive an input, via a user interface, indicating an updated sleep-threshold amount of time that corresponds to the expiry date of the particular therapeutic substance being used with therapeutic substance delivery apparatus 20. Examples of the user interface may be a control panel on therapeutic substance delivery apparatus 20 itself, or an application on a wireless device such as a phone or tablet.

In order to account for tolerances within therapeutic substance delivery apparatus 20, e.g., tolerances in the sensitivity of position sensor 30, and tolerances in position and size of plunger-sensor engagement interface 28, plunger 26 and position sensor 30 are arranged such that as plunger 26 slides distally in response to filling, there is a distance D1 that plunger 26 must traverse before plunger-sensor engagement interface 28 causes position sensor 30 to switch states from S₁ to S₂. As such, as plunger 26 slides distally from initial proximal position P1, an initial volume of the therapeutic substance enters reservoir 22 prior to plunger-sensor engagement interface 28 causing position sensor 30 to change state from S₁ to S₂. Distance D1 thus prevents therapeutic substance delivery apparatus 20 from accidentally waking up before reservoir 22 is actually being filled with the therapeutic substance.

Distance D1 is depicted in segment (3-4) of Fig. 1A as the distance between initial proximal position P1 and intermediate position P2 (at which plunger-sensor engagement interface 28 causes position sensor 30 to switch states from S₁ to S₂). For some applications, distance D1 is at least 0.5 mm, i.e., plunger 26 and position sensor 30 are arranged such that distal movement of plunger 26 from initial proximal position P1 less than 0.5 mm toward intermediate position P2 does not cause plunger-sensor engagement interface 28 to change the state of position sensor 30 from S₁ to S₂.

Reference is now made specifically to segments (3-4) and (5) of Fig. 1A, and to lines 4-5 of the state table of Fig. 3, which correspond to control circuitry 32 monitoring the filling processes of reservoir 22 in order to determine when reservoir 22 has been sufficiently filled. It is noted that for some applications, the user may be provided with a syringe that is prefilled with the exact amount of therapeutic substance that is needed, along with instructions to fill reservoir 22 with the entire contents of the syringe. In this case, control circuitry 32 does not monitor therapeutic substance delivery apparatus 20 for sufficient filling. Rather, subsequently to therapeutic substance delivery apparatus 20 waking up in response to the filling of reservoir 22, control circuitry 32 initiates the delivery process in which control circuitry 32 drives therapeutic substance delivery apparatus 20 to deliver the therapeutic substance from reservoir 22 to the subject by driving plunger 26 to slide proximally within reservoir 22.

For some applications, control circuitry 32 monitors the filling of reservoir 22 so as to identify when therapeutic substance delivery apparatus 20 has reached a filling-complete state. In this case, plunger 26 and position sensor 30 are arranged such that distal movement of plunger 26 past intermediate position P2 to a post-intermediate position P3 within reservoir 22 causes plunger-sensor engagement interface 28 to change the state of position sensor 30 from S₂ back to S₁. Post-intermediate position P3 is illustrated by the dashed line labeled P3 spanning segments (3-4) and (5) of Fig. 1A. Distal movement of plunger 26 from intermediate position P2 to post-intermediate position P3 corresponds to the completion of reservoir 22 being filled with a threshold volume of the therapeutic substance (further described hereinbelow with reference to Fig. 2). That is, upon completion of reservoir 22 being filled with at least a threshold volume of the therapeutic substance, plunger-sensor engagement interface 28 causes position sensor 30 to change state from S₂ back to S₁. Control circuitry 32 is configured to identify that therapeutic substance delivery apparatus 20 is in a filling-complete state in response to position sensor 30 changing state from S₂ to S₁. This corresponds to line 5 of the state table of Fig. 3 and is depicted by the transition from segment (3-4) to segment (5) of Fig. 1A. Under normal operating conditions, i.e., in the absence of any error on the part of the user or the apparatus, filling reservoir 22 with the threshold volume of the therapeutic substance is the only time during operation of therapeutic substance delivery apparatus 20 that position sensor 30 switches states from S₂ to S₁.

For some applications, plunger 26 and position sensor 30 are arranged such that, subsequently to plunger-sensor engagement interface 28 causing position sensor 30 to change state from S₁ to S₂ (i.e., waking up therapeutic substance delivery apparatus 20), plunger-sensor engagement interface 28 maintains engagement with position sensor 30, thereby maintaining position sensor 30 in state S₂, until reservoir 22 has been filled with the threshold volume of the therapeutic substance. Further distal movement of plunger 26 subsequently to the reservoir being filled with the threshold volume of the therapeutic substance, i.e., plunger 26 reaching post-intermediate position P3, causes plunger-sensor engagement interface 28 to terminate engagement with position sensor 30, causing position sensor 30 to switch states from S₂ back to S₁.

For some applications, a dimension of plunger-sensor engagement interface 28 determines how long plunger-sensor engagement interface 28 maintains engagement with position sensor 30, i.e., a distance D3 between two fixed points on plunger-sensor engagement interface 28 determines when plunger-sensor engagement interface 28 terminates engagement with position sensor 30. In order to ensure that upon completion of filling reservoir 22 with the threshold volume of the therapeutic substance position sensor 30 does indeed switch states from S₂ back to S₁, tolerances within therapeutic substance delivery apparatus 20 should be accounted for, e.g., tolerances in the sensitivity of the microswitch. This is achieved by a distance D2 between intermediate position P2 and post-intermediate position P3 being large enough, e.g., at least 0.1 mm, so as to ensure that plunger-sensor engagement interface 28 terminates engagement with position sensor 30 upon plunger 26 reaching post-intermediate position P3. This is depicted as well by dashed line 36 spanning segments (3-4) and (5) of Fig. 1A. Plunger-sensor engagement interface 28 maintains engagement with position sensor 30 until the entire distance D3 between the two fixed points on plunger-sensor engagement interface 28 clears the height of dashed line 36, as shown in segment (5) of Fig. 1A, at which point plunger-sensor engagement interface 28 terminates engagement with position sensor 30 allowing position sensor 30 to return to state S₁.

In the event that a filling error occurs, e.g., plunger 26 gets stuck and is unable to move distally in order to accommodate the incoming therapeutic substance, or the user stops filling in the middle, control circuitry 32 is able to identify that a filling error has occurred in response to (i) the state of therapeutic substance delivery apparatus 20 being filling (as previously identified by the switch of position sensor 30 from S₁ to S₂), in combination with (ii) a filling-threshold amount of time having elapsed from when position sensor 30 changes state from S₁ to S₂ without position sensor 30 changing state from S₂ back to S₁. This corresponds to line 4 of the state table of Fig. 3, and is depicted by segment (3-4) of Fig. 1A, i.e., therapeutic substance delivery apparatus 20 remaining in the state depicted in segment (3-4) of Fig. 1A for longer than the filling-threshold amount of time. For example, if it is expected that filling reservoir 22 with the therapeutic substance should take a certain amount of time t1, then the filling-threshold amount of time is set as an amount of time t2 that is larger than t1. An additional type of filling error that may occur is the user erroneously withdrawing some of therapeutic substance back into the syringe before position sensor changes state from S₂ back to S₁.

For some applications, in the event of a filling error, therapeutic substance delivery apparatus 20 provides an alert and then disables itself such that delivery of the therapeutic substance does not begin. Alternatively or additionally, for some applications, in the event of a filling error, therapeutic substance delivery apparatus 20 provides an alert to the user that filling of the reservoir is not complete and allows the user to resume filling reservoir 22. Subsequently, if the fill-threshold amount of time (or another amount of time) elapses again without position sensor 30 changing state from S₂ back to S₁, therapeutic substance delivery apparatus 20 disables itself.

Reference is now made specifically to segment (6) of Fig. 1A, Fig. 1B, and lines 6-10 of the state table of Fig. 3. Plunger 26 and position sensor 30 are arranged such that, subsequently to position sensor 30 changing state from S₂ back to S₁, proximal movement of plunger 26 within reservoir 22 causes plunger-sensor engagement interface 28 to change the state of position sensor 30 from S₁ to the third state S₃. Proximal motion of plunger 26, occurring subsequently to reservoir 22 being filled with at least the threshold volume of therapeutic substance, occurs as therapeutic substance leaves reservoir 22. Typically, therapeutic substance leaves reservoir 22 in any of the following scenarios, each further described hereinbelow: (a) during delivery of the therapeutic substance from reservoir 22 to the subject, (b) in the event of a leak in reservoir 22, or (c) therapeutic substance erroneously being drawn out of reservoir 22 back into the syringe.

Typically, as described hereinabove, upon completion of reservoir 22 being filled with at least the threshold volume of the therapeutic substance, therapeutic substance delivery apparatus 20 is in a filling-complete state, and position sensor 30 is in state S₁, as depicted in segment (7) of Fig. 1B. Subsequently to therapeutic substance delivery apparatus 20 being in the filling-complete state, control circuitry 32 is configured to initiate a delivery-started state of the apparatus in which control circuitry 32 drives therapeutic substance delivery apparatus 20 to deliver the therapeutic substance from reservoir 22 to the subject by driving plunger 26 to slide proximally within reservoir 22. Plunger 26 and position sensor 30 are arranged such that as plunger 26 slides proximally during the delivery of the therapeutic substance to the subject, plunger-sensor engagement interface 28 causes position sensor 30 to change state from S₁ to S₃.

As illustrated in segment (5) of Fig. 1A, due to plunger 26 having moved distally to at least post-intermediate position P3 during filling, upon completion of the filling of reservoir 22 with the threshold amount of therapeutic substance, plunger-sensor engagement interface 28 is positioned distal to position sensor 30 by at least a distance D4. When control circuitry 32 initiates the delivery-started state a certain amount of wait-time is expected to elapse while plunger-sensor engagement interface 28 traverses at least distance D4 in the proximal direction before plunger-sensor engagement interface 28 engages with position sensor 30 in order to switch it to S₃. As further described hereinbelow with reference to Fig. 2, depending on an actual volume of therapeutic substance that is filled into reservoir 22, e.g., a volume greater than the threshold volume, the expected wait-time until plunger-sensor engagement interface 28 engages with position sensor 30 in order to switch it to state S₃ may be different than the minimum expected-wait time associated with distance D4.

Control circuitry 32 is configured to identify that therapeutic substance delivery apparatus 20 is in a delivery-in-progress state, in which therapeutic substance is being delivered from reservoir 22 to the subject, in response to position sensor 30 changing state from S₁ to S₃ subsequently to control circuitry 32 initiating the delivery-started state of therapeutic substance delivery apparatus 20 and the expected wait-time having elapsed. This corresponds to line 8 of the state table of Fig. 3 and is depicted by the transition from segment (7) to segment (8-9) of Fig. 1B.

Typically, part of the process of initiating the delivery-started state of therapeutic substance delivery apparatus 20 is to fluidly connect reservoir 22 to the fluid path through which the therapeutic substance is delivered to the subject. This is done by control circuitry 32 activating a needle slider to drive a fluid path needle 38 into the proximal end of reservoir 22 (e.g., using techniques and apparatus described in US Patent Application Publication US 2020/0108201 to Ben David et al., which is incorporated herein by reference). As shown in Fig. 1A, in which all the segments correspond to states of therapeutic substance delivery apparatus 20 that occur prior to initiation of the delivery-started state, fluid path needle 38 is disposed outside of reservoir 22 and as such reservoir 22 is not in fluid communication with the subject. As shown in Fig. 1B, in which all the segments correspond to states of therapeutic substance delivery apparatus 20 after control circuitry 32 has initiated the delivery-started state, fluid path needle 38 is disposed within reservoir 22.

As described hereinabove, one undesired way in which therapeutic substance may leave reservoir 22 subsequently to reservoir 22 having been filled is if there is a leak in reservoir 22. Control circuitry 32 is configured to identify that there is a leak in reservoir 22 in response to position sensor 30 changing state from S₁ to S₃ prior to control circuitry 32 initiating the delivery-started state of therapeutic substance delivery apparatus 20. Typically, in response to the identification that there is a leak, control circuitry 32 disables therapeutic substance delivery apparatus 20. This corresponds to line 6 of the state table of Fig. 3, and is depicted in segment (6) of Fig. 1A, in which plunger-sensor engagement interface 28 has engaged position sensor 30 causing it to change state to S₃ while fluid path needle 38 still remains outside of reservoir 22, indicating that the delivery-started state has not yet been initiated by control circuitry 32. It is noted that if some or all of the therapeutic substance is erroneously drawn back out of reservoir 22 via the filling port and into the syringe subsequently to the reservoir 22 having been filled with the threshold volume of therapeutic substance, it is identified as a leak by control circuitry 32.

As described hereinabove, post-intermediate position P3 corresponds to the threshold volume of therapeutic substance that has to be filled within reservoir 22 in order for control circuitry 32 to identify that therapeutic substance delivery apparatus 20 is in a filling-complete state. However, for many therapeutic substances, different patients will be prescribed different dosages of therapeutic substance to be delivered, e.g., based on a subject's weight, or severity of condition. Typically, therapeutic substance delivery apparatus 20 is configured such that the threshold volume of therapeutic substance needed in order for control circuitry 32 to identify the filling-complete state corresponds to a minimum reasonable dosage that may be prescribed for a given therapeutic substance being administered by therapeutic substance delivery apparatus 20. At the manufacturing stage, therapeutic substance delivery apparatus 20 may be configured for a specific therapeutic substance. For example, distance D3, which determines when plunger-sensor engagement interface 28 terminates engagement with position sensor 30 during filling, may be configured to correspond to a specific threshold volume for a particular therapeutic substance.

Segment (7) of Fig. 2 depicts a scenario in which reservoir 22 has been filled with the threshold volume of therapeutic substance. This position of plunger 26 is represented by dashed line 40 spanning all the segments of Fig. 2, and is the same position as post-intermediate position P3. As described hereinabove with reference to segment (5) of Fig. 1A, distance D4 corresponds to the distance that plunger 26 has to traverse in the proximal direction before plunger-sensor engagement interface 28 causes position sensor 30 to change state from S₁ to S₃. Distance D4 also defines the expected wait-time before control circuitry 32 expects to receive an input that position sensor 30 has changed state from S₁ to S₃, indicating that delivery has started.

For some applications, a given subject may be prescribed a volume of therapeutic substance that is larger than the threshold volume. For some applications, therapeutic substance delivery apparatus 20 is packaged for commercial sale including a syringe that holds a standard volume of therapeutic substance, e.g., 10 ml, regardless of how much therapeutic substance a particular subject is supposed to receive. If a given subject is prescribed a volume of therapeutic substance that is larger than the threshold volume, then upon completion of the subject filling reservoir 22 with the prescribed volume, plunger-sensor engagement interface 28 is positioned distal to position sensor 30 by a distance that is larger than D4. As such, plunger 26 has to traverse this larger distance in the proximal direction before plunger-sensor engagement interface 28 causes position sensor 30 to change states from S₁ to S₃. Therefore, the inventors have realized that in order to accommodate therapeutic substance delivery apparatus 20 being usable with various different dosages of a therapeutic substance, control circuitry 32 is configured to (a) receive an input indicating an intended volume of the therapeutic substance to be filled within reservoir 22, and (b) in response to the received input, determine the length of the expected wait-time between control circuitry 32 initiating the delivery-started state of the apparatus and plunger-sensor engagement interface 28 causing position sensor 30 to change state from S₁ to S₃.

For some applications, control circuitry 32 receives the input indicating the intended volume of therapeutic substance to be filled within reservoir 22 during the manufacturing processes of therapeutic substance delivery apparatus 20. For example, software may be downloaded to therapeutic substance delivery apparatus 20 to preprogram therapeutic substance delivery apparatus 20 for a specific intended volume of therapeutic substance. In this case, there may be several different configurations of therapeutic substance delivery apparatus 20 that are sold commercially, and a subject will be prescribed a device that corresponds to a specific prescribed dosage of therapeutic substance.

Additionally or alternatively, therapeutic substance delivery apparatus 20 may be sold having a certain configuration, e.g., preconfigured for the threshold volume of therapeutic substance, and may then be reconfigured via a user interface through which control circuitry 32 receives the input indicating the intended volume of therapeutic substance. For example, the user, e.g., the subject who receives the therapeutic substance, a pharmacist, or caregiver, may configure the device according to the prescribed amount of therapeutic substance. Examples of the user interface may be a control panel on therapeutic substance delivery apparatus 20 itself, or an application on a wireless device such as a phone or tablet.

Segment (7') in Fig. 2 depicts the position of plunger 26 after reservoir 22 has been filled with an intended volume of therapeutic substance that is larger than the threshold volume of therapeutic substance. This position of plunger 26 is represented by dashed line 42 spanning all the segments of Fig. 2. Distance D5 corresponds to the distance that plunger 26 has to traverse in the proximal direction during delivery of the therapeutic substance prior to plunger-sensor engagement interface 28 causing position sensor 30 to change state from S₁ to S₃. As described hereinabove, D5 is larger than D4 due to the intended volume of therapeutic substance being larger than the threshold volume of therapeutic substance. Due to control circuitry 32 having received an input indicating the intended volume of therapeutic substance, control circuitry 32 calculates an expected wait-time that corresponds to distance D5. Thus, control circuitry 32 is configured to identify that therapeutic substance delivery apparatus 20 is in a delivery-in-progress state in response to position sensor 30 changing state from S₁ to S₃ subsequently to control circuitry 32 initiating the delivery-started state of therapeutic substance delivery apparatus 20 and the expected wait-time corresponding to distance D5 having elapsed.

Due to reservoir 22 being filled with therapeutic substance by a user of therapeutic substance delivery apparatus 20, e.g., the subject, a pharmacist, or a caregiver, there is a margin for human error. For example, it is possible that a user may accidentally fill reservoir 22 with a volume of therapeutic substance that surpasses the threshold volume, but that is less than or greater than the intended volume. In this case, due to plunger 26 having reached post-intermediate position P3 (once the threshold volume has been filled), control circuitry 32 identifies that filling is complete (line 5 of the state table) and may initiate the delivery-started state of therapeutic substance delivery apparatus 20 despite the wrong volume of therapeutic substance having been filled within reservoir 22. Additionally or alternatively, if reservoir 22 has a leak in it then even if the user correctly fills reservoir 22 with the intended volume, some therapeutic substance may exit reservoir 22 causing plunger 26 to slide proximally within reservoir 22.

The inventors have realized that control circuitry 32 having received the input indicating the intended volume, and having determined a corresponding expected wait time, allows control circuitry 32 to identify if reservoir 22 is not holding the correct, i.e., intended, volume of therapeutic substance. Position sensor 30 is expected to switch states from S₁ to S₃ after the expected wait-time corresponding to distance D5 has elapsed. As further described hereinbelow with reference to segments (7") and (7‴) of Fig. 2, position sensor 30 switching states from S₁ to S₃ after an amount of time that is either less than or greater than the expected wait-time is an indication that reservoir 22 was not holding the correct, i.e., intended, volume of therapeutic substance.

Segment (7") of Fig. 2 depicts an example in which either the user has underfilled reservoir 22, i.e., has filled reservoir 22 with a volume of therapeutic substance that is less than the intended volume, or a leak in reservoir 22 has caused plunger 26 to slide proximally after the user filled reservoir 22 with the intended volume. This position of plunger 26 is represented by dashed line 44. Distance D6 represents the actual distance plunger 26 traverses in this case prior to causing position sensor 30 to change states from S₁ to S₃. As can be seen, distance D6 is less than distance D5, resulting in plunger-sensor engagement interface 28 causing position sensor 30 to change states from S₁ to S₃ prior to the expected wait-time (corresponding to distance D5) having elapsed. Regardless of whether this is caused by the user underfilling reservoir 22 or a leak in reservoir 22, therapeutic substance delivery apparatus 20 is not able to deliver the intended volume of therapeutic substance to the subject. As such, control circuitry 32 is configured to terminate the delivery of the therapeutic substance to the subject in response to (i) the current state of the apparatus being delivery-started, in combination with (ii) position sensor 30 changing state from S₁ to S₃ prior to the expected wait-time having elapsed. Alternatively, control circuitry 32 may continue the delivery of the therapeutic substance to the subject and generate an alert indicating to the subject that the full amount of the intended volume has not been delivered to the subject.

Segment (7‴) of Fig. 2 depicts an example in which the user has overfilled reservoir 22, i.e., has filled reservoir 22 with a volume of therapeutic substance that is larger than the intended volume. This position of plunger 26 is represented by dashed line 46. Distance D7 represents the actual distance plunger 26 traverses in this case prior to causing position sensor 30 to change states from S₁ to S₃. As can be seen, distance D6 is larger than distance D5, resulting in plunger-sensor engagement interface 28 causing position sensor 30 to change states from S₁ to S₃ after an amount of time that is longer than the expected wait-time (corresponding to distance D5) but shorter than a delivery-start-threshold amount of time (further described hereinbelow with reference to line 7 of the state table of Fig. 3). Thus, control circuitry 32 is configured to identify that reservoir 22 has been filled with a volume of therapeutic substance that is larger than the intended volume in response to (i) the current state of the apparatus being delivery-started, in combination with (ii) position sensor 30 changing state from S₁ to S₃ after an amount of time that is longer than the expected wait-time but shorter than a delivery-start-threshold amount of time.

For some applications, control circuitry 32 terminates delivery of the therapeutic substance to the subject in response to reservoir 22 having been filled with the volume of therapeutic substance that is larger than the intended volume. Alternatively, control circuitry 32 may compensate for the difference in volume between the intended volume and the larger volume by terminating the delivery of the therapeutic substance upon delivering the intended volume to the subject, notwithstanding there being extra therapeutic substance within reservoir 22. Typically, delivery of the therapeutic substance to the subject is driven by an electromechanical pumping assembly (e.g., using techniques and apparatus described in US Patent Application Publications US 2019/0365985 to Zidon et al. and US 2020/0108201 to Ben David et al., which are incorporated herein by reference). The electromechanical pumping assembly typically includes a motor, e.g., a stepper motor, and control circuitry 32 is able to monitor how much therapeutic substance has been delivered to the subject by monitoring the pumping cycles of the electromechanical pumping assembly, e.g., by monitoring the motor. This allows control circuitry 32 to terminate the delivery upon the intended volume of therapeutic substance having been delivered to the subject.

Typically, under normal operating conditions where reservoir 22 is filled with the intended volume of therapeutic substance, plunger 26 and position sensor 30 are arranged such that upon completion of delivery of the intended volume of therapeutic substance from reservoir 22, plunger-sensor engagement interface 28 causes the position sensor 30 to change state from S₃ back to S₁. Control circuitry 32 identifies that therapeutic substance delivery apparatus 20 is in a delivery-complete state in response to the position sensor changing state from Sato S₁. This corresponds to line 10 of the state table of Fig. 3, and is depicted in segment (10) of Fig. 1B. Typically, completion of the delivery of the therapeutic substance is the only time during operation of therapeutic substance delivery apparatus 20 that position sensor 30 switches states from S₃ to S₁.

In the above example in which (a) reservoir 22 was filled with a volume of therapeutic substance that is larger than the intended volume and (b) control circuitry 32 compensates for the difference by terminating the delivery upon the intended volume of therapeutic substance having been delivered to the subject, control circuitry 32 terminates the delivery of the therapeutic substance to the subject notwithstanding position sensor 30 remaining in state S₃.

Reference is now made specifically to lines 7 of the state table of Fig. 3. For some applications, another reason that an amount of time that is longer than the expected wait-time may have elapsed from when control circuitry 32 initiates the delivery-started state, is that an internal error has occurred within therapeutic substance delivery apparatus 20. For example, delivery of the therapeutic substance may not have started in response to control circuitry 32 initiating the delivery-started state, or plunger 26 may be stuck and unable to slide within reservoir 22. As such, a delivery-start-threshold amount of time is set such that control circuitry 32 identifies that a delivery-start error has occurred in response to (i) the current state of therapeutic substance delivery apparatus 20 being delivery-started (with position sensor 30 being in state S₁), in combination with (ii) the delivery-start-threshold amount of time having elapsed from when control circuitry 32 initiated the delivery-started state without position sensor 30 changing state from S₁ to S₃. If the delivery-start-threshold amount of time elapses, it is typically too long an amount of time to be considered as reservoir 22 having been overfilled. Typically, in response to identifying that a delivery-start error has occurred, control circuitry 32 disables therapeutic substance delivery apparatus 20.

Reference is now made specifically to line 9 of the state table of Fig. 3. Another type of error which may occur during operation of therapeutic substance delivery apparatus 20 is a delivery-progress error, i.e., delivery of the therapeutic substance is interrupted and does not reach completion. For example, plunger 26 may get stuck within reservoir 22, or an internal error in therapeutic substance delivery apparatus 20 may cause the delivery of the therapeutic substance to stop mid-delivery. As described hereinabove, control circuitry 32 identifies that the delivery of the therapeutic substance is complete in response to position sensor 30 changing state from S₃ back to S₁ after delivery has begun. If a delivery-progress error occurs such that the delivery is interrupted, position sensor 30 does not change state from S₃ back to S₁. Thus, control circuitry 32 is configured to identify that a delivery-progress error has occurred in response to (i) the current state of therapeutic substance delivery apparatus 20 being delivery-in-progress, in combination with (ii) a delivery-progress-threshold amount of time having elapsed from when position sensor 30 changes state from S₁ to S₃ (indicating that delivery is in progress) without position sensor 30 changing state from S₃ back to S₁.

Applications of the invention described herein can take the form of a computer program product accessible from a computer-usable or computer-readable medium (e.g., a non-transitory computer-readable medium) providing program code for use by or in connection with a computer or any instruction execution system, such as control circuitry 32. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W) and DVD. For some applications, cloud storage, and/or storage in a remote server is used.

A data processing system suitable for storing and/or executing program code will include at least one processor (e.g., control circuitry 32) coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an objectoriented programming language such as Java, Smalltalk, C++ or the like and conventional procedural programming languages, such as the C programming language or similar programming languages.

It will be understood that the methods described herein can be implemented by computer program instructions. These computer program instructions may be provided to a processor of a general-purpose computer, special purpose computer, or other programmable data processing apparatus to produce a machine, such that the instructions, which execute via the processor of the computer (e.g., control circuitry 32) or other programmable data processing apparatus, create means for implementing the functions/acts specified in the methods described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the methods described in the present application. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the methods described in the present application.

Control circuitry 32 is typically a hardware device programmed with computer program instructions to produce a special purpose computer. For example, when programmed to perform the methods described herein, the computer processor typically acts as a special purpose computer processor. Typically, the operations described herein that are performed by computer processors transform the physical state of a memory, which is a real physical article, to have a different magnetic polarity, electrical charge, or the like depending on the technology of the memory that is used.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. A therapeutic substance delivery apparatus (20) comprising:
a housing (24);
a reservoir (22) disposed at least partially within the housing (24) and configured to hold a therapeutic substance;
a position sensor (30) having three discrete states (S₁, S₂, S₃);
a plunger (26), slidable within the reservoir (22);
a plunger-sensor engagement interface (28) that is coupled to the plunger (26) within the reservoir (22) and extends from within the reservoir (22) to outside of the reservoir (22), wherein the plunger (26) and the position sensor (30) are arranged such that the plunger-sensor engagement interface (28) causes the position sensor (30) to change states as the plunger (26) slides within the reservoir (22); and
control circuitry (32) configured to identify a new state indication of the apparatus (20) based on (a) a change in state of the position sensor (30), or (b) a previous or current state of the apparatus (20) in combination with a threshold amount of time having elapsed without the position sensor (30) changing state,
wherein:
the plunger (26) and the position sensor (30) are arranged such that distal movement of the plunger (26) from an initial proximal position within the reservoir (22) to an intermediate position within the reservoir (22) causes the plunger-sensor engagement interface (28) to change the state of the position sensor (30) from a first state S₁ of the discrete states in which the plunger-sensor engagement interface (28) is not engaged with the position sensor (30) to a second state S₂ of the discrete states in which the plunger-sensor engagement interface (28) is engaged with the position sensor (30) as the plunger (26) slides distally within the reservoir (22),
the plunger (26) and the position sensor (30) are arranged such that distal movement of the plunger (26) past the intermediate position to a post-intermediate position within the reservoir (22) causes the plunger-sensor engagement interface (28) to change the state of the position sensor (30) from Sz back to S₁,
the plunger (26) and the position sensor (30) are arranged such that, subsequently to the position sensor (30) changing state from Sz back to S₁, proximal movement of the plunger (26) within the reservoir (22) causes the plunger-sensor engagement interface (28) to change the state of the position sensor (30) from S₁ to a third state S₃ of the discrete states in which the plunger-sensor engagement interface (28) is engaged with the position sensor (30) as the plunger (26) slides proximally within the reservoir (22), and
the plunger (26) and the position sensor (30) are arranged such that, subsequently to the position sensor (30) changing from S₁ to S₃, further proximal movement of the plunger (26) causes the plunger-sensor engagement interface (28) to change the state of the position sensor (30) from S₃ back to S₁.

2. The apparatus (20) according to claim 1, wherein the position sensor (30) has exactly three discrete states (S₁, S₂, S₃).

3. The apparatus (20) according to any one of claims 1-2, wherein:
(a) the housing (24) is shaped to define a filling port (34) in fluid communication with the reservoir (22), and wherein the reservoir (22) is configured to be filled with the therapeutic substance via the filling port (34),
(b) prior to the filling of the reservoir (22) the apparatus (20) is in a sleep state, the plunger (26) is disposed at an initial proximal position within the reservoir (22), and the position sensor (30) is in state S₁,
(c) filling the reservoir (22) with the therapeutic substance causes the plunger (26) to slide in a distal direction from the initial proximal position,
(d) the plunger (26) and the position sensor (30) are arranged such that as the plunger (26) slides in the distal direction, the plunger-sensor engagement interface (28) causes the position sensor (30) to change state from S₁ to S₂, and
(e) the control circuitry (32) is configured to identify that the apparatus (20) is awake and in a filling state in response to the position sensor (30) changing state from S₁ to S₂.

4. The apparatus (20) according to claim 3, wherein:
the plunger (26) and the position sensor (30) are arranged such that upon completion of the reservoir (22) being filled with a threshold volume of the therapeutic substance, the plunger-sensor engagement interface (28) causes the position sensor (30) to change state from Sz back to S₁, and
the control circuitry (32) is configured to identify that a filling error has occurred in response to (i) the state of the apparatus (20) being filling, in combination with (ii) a filling-threshold amount of time having elapsed from when the position sensor (30) changes state from S₁ to S₂ without the position sensor (30) changing state from Sz back to S₁.

5. The apparatus (20) according to claim 3, wherein the plunger (26) and the position sensor (30) are arranged such that upon completion of the reservoir (22) being filled with at least a threshold volume of the therapeutic substance, the plunger-sensor engagement interface (28) causes the position sensor (30) to change state from Sz back to S₁, and wherein the control circuitry (32) is configured to identify that the apparatus (20) is in a filling-complete state in response to the position sensor (30) changing state from Sz back to S₁.

6. The apparatus (20) according to claim 5, wherein:
the plunger (26) and the position sensor (30) are arranged such that, subsequently to the plunger-sensor engagement interface (28) causing the position sensor (30) to change state from S₁ to S₂, the plunger-sensor engagement interface (28) maintains engagement with the position sensor (30), so as to maintain the position sensor (30) in state S₂, until the reservoir (22) has been filled with the threshold volume of the therapeutic substance, and
the plunger (26) and the position sensor (30) are arranged such that further distal movement of the plunger (26) subsequently to the reservoir (22) being filled with the threshold volume of the therapeutic substance causes the plunger-sensor engagement interface (28) to terminate engagement with the position sensor (30), causing the position sensor (30) to switch from Sz back to S₁.

7. The apparatus (20) according to claim 3, wherein:
(a) upon completion of the reservoir (22) being filled with at least a threshold volume of the therapeutic substance, the apparatus (20) is in a filling-complete state, and the position sensor (30) is in state S₁,
(b) subsequently to the apparatus (20) being in the filling-complete state, the control circuitry (32) is configured to initiate a delivery-started state of the apparatus (20) in which the control circuitry (32) drives the therapeutic substance delivery apparatus (20) to deliver the therapeutic substance from the reservoir (22) to a subject by driving the plunger (26) to slide proximally within the reservoir (22), and
(c) the plunger (26) and the position sensor (30) are arranged such that as the plunger (26) slides proximally the plunger-sensor engagement interface (28) causes the position sensor (30) to change state from S₁ to S₃.

8. The apparatus (20) according to claim 7, wherein the control circuitry (32) is further configured to (a) receive an input indicating an intended volume of the therapeutic substance to be filled within the reservoir (22), and (b) in response to the received input, determine the length of an expected wait-time between the control circuitry (32) initiating the delivery-started state of the apparatus (20) and the plunger-sensor engagement interface (28) causing the position sensor (30) to change state from S₁ to S₃.

9. The apparatus (20) according to claim 8, wherein:
(a) the control circuitry (32) is configured to identify that the reservoir (22) has been filled with a volume of therapeutic substance that is larger than the intended volume in response to (i) the current state of the apparatus (20) being delivery-started, in combination with (ii) the position sensor (30) changing state from S₁ to S₃ after an amount of time that is longer than the expected wait-time but shorter than a delivery-start-threshold amount of time,
(b) the control circuitry (32) is configured to compensate for the difference in volume between the intended volume and the volume of therapeutic substance within the reservoir (22) that is larger than the intended volume by terminating the delivery of the therapeutic substance to the subject upon delivering the intended volume to the subject,
(c) the plunger (26) and the position sensor (30) are arranged such that, in response to the reservoir (22) being filled with the intended volume, upon delivering the intended volume to the subject the plunger-sensor engagement interface (28) causes the position sensor (30) to change state from S₃ back to S₁, and
(d) in response to the identification that the reservoir (22) has been filled with a volume of therapeutic substance that is larger than the intended volume, upon delivering the intended volume to the subject, the control circuitry (32) is configured to terminate the delivery of the therapeutic substance to the subject notwithstanding the position sensor (30) remaining in state S₃.

10. The apparatus (20) according to claim 8, wherein the control circuitry (32) is configured to identify that the apparatus (20) is in a delivery-in-progress state, in which therapeutic substance is being delivered from the reservoir (22) to the subject, in response to the position sensor (30) changing state from S₁ to S₃ subsequently to the control circuitry (32) initiating the delivery-started state of the apparatus (20) and the expected wait-time having elapsed.

11. The apparatus (20) according to claim 10, wherein the plunger (26) and the position sensor (30) are arranged such that upon completion of delivery of the intended volume of therapeutic substance from the reservoir (22), the plunger-sensor engagement interface (28) causes the position sensor (30) to change state from S₃ back to S₁, and wherein the control circuitry (32) is configured to identify that the apparatus (20) is in a delivery-complete state in response to the position sensor (30) changing state from S₃ to S₁.

12. The apparatus (20) according to claim 11, wherein the control circuitry (32) is configured to identify that a delivery-progress error has occurred in response to (i) the current state of the apparatus (20) being delivery-in-progress, in combination with (ii) a delivery-progress-threshold amount of time having elapsed from when the position sensor (30) changes state from S₁ to S₃ without the position sensor (30) changing state from S₃ back to S₁.

13. The apparatus (20) according to any one of claims 1-2, wherein the apparatus (20) is configured such that when the reservoir (22) is in a filled state in which a volume of therapeutic substance is disposed within the reservoir (22) and the position sensor (30) is in state S₁:
(a) the control circuitry (32) is configured to initiate a delivery-started state of the apparatus (20) in which the control circuitry (32) drives the therapeutic substance delivery apparatus (20) to deliver the therapeutic substance from the reservoir (22) to a subject by driving the plunger (26) to slide proximally within the reservoir (22), and
(b) the plunger (26) and the position sensor (30) are arranged such that as the plunger (26) slides proximally, the plunger-sensor engagement interface (28) causes the position sensor (30) to change state from S₁ to a post-first state.

## Patentansprüche

1. Abgabeeinrichtung (20) für eine therapeutische Substanz, umfassend:
ein Gehäuse (24);
ein Reservoir (22), das wenigstens teilweise innerhalb des Gehäuses (24) angeordnet und dazu konfiguriert ist, eine therapeutische Substanz zu enthalten;
einen Positionssensor (30) mit drei diskreten Zuständen (S₁, S₂, S₃);
einen Kolben (26), der in dem Reservoir (22) gleitbar ist;
eine Kolben-Sensor-Eingriffsschnittstelle (28), die mit dem Kolben (26) innerhalb des Reservoirs (22) gekoppelt ist und sich von innerhalb des Reservoirs (22) nach außerhalb des Reservoirs (22) erstreckt, wobei der Kolben (26) und der Positionssensor (30) derart angeordnet sind, dass die Kolben-Sensor-Eingriffsschnittstelle (28) den Positionssensor (30) veranlasst, seinen Zustand zu ändern, wenn der Kolben (26) innerhalb des Reservoirs (22) gleitet; und
eine Steuerschaltung (32), die dazu konfiguriert ist, eine neue Zustandsanzeige der Einrichtung (20) auf Grundlage (a) einer Zustandsänderung des Positionssensors (30) oder (b) eines vorherigen oder aktuellen Zustands der Einrichtung (20) in Kombination mit einer Schwellenzeitspanne, die verstrichen ist, ohne dass der Positionssensor (30) den Zustand ändert, zu identifizieren,
wobei:
der Kolben (26) und der Positionssensor (30) derart angeordnet sind, dass eine distale Bewegung des Kolbens (26) von einer anfänglichen proximalen Position innerhalb des Reservoirs (22) zu einer Zwischenposition innerhalb des Reservoirs (22) veranlasst, dass die Kolben-Sensor-Eingriffsschnittstelle (28) den Zustand des Positionssensors (30) von einem ersten Zustand S₁ der diskreten Zustände, in dem die Kolben-Sensor-Eingriffsschnittstelle (28) nicht mit dem Positionssensor (30) in Eingriff steht, zu einem zweiten Zustand S₂ der diskreten Zustände zu ändern, in dem die Kolben-Sensor-Eingriffsschnittstelle (28) mit dem Positionssensor (30) in Eingriff steht, wenn der Kolben (26) innerhalb des Reservoirs (22) distal gleitet,
der Kolben (26) und der Positionssensor (30) derart angeordnet sind, dass eine distale Bewegung des Kolbens (26) an der Zwischenposition vorbei zu einer Post-Zwischenposition innerhalb des Reservoirs (22) veranlasst, dass die Kolben-Sensor-Eingriffsschnittstelle (28) den Zustand des Positionssensors (30) von S₂ zurück zu S₁ ändert,
der Kolben (26) und der Positionssensor (30) derart angeordnet sind, dass, nachdem der Positionssensor (30) den Zustand von S₂ zurück zu S₁ geändert hat, eine proximale Bewegung des Kolbens (26) innerhalb des Reservoirs (22) veranlasst, dass die Kolben-Sensor-Eingriffsschnittstelle (28) den Zustand des Positionssensors (30) von S₁ in einen dritten Zustand S₃ der diskreten Zustände ändert, in dem die Kolben-Sensor-Eingriffsschnittstelle mit dem Positionssensor (30) in Eingriff steht, wenn der Kolben (26) innerhalb des Reservoirs (22) proximal gleitet, und
der Kolben (26) und der Positionssensor (30) derart angeordnet sind, dass nachdem der Positionssensor (30) den Zustand von S₁ zu S₃ geändert hat, eine weitere proximale Bewegung des Kolbens (26) veranlasst, dass die Kolben-Sensor-Eingriffsschnittstelle (28) den Zustand des Positionssensors (30) von S₃ zurück zu S₁ ändert.

2. Einrichtung (20) nach Anspruch 1, wobei der Positionssensor (30) genau drei diskrete Zustände (S₁, S₂, S₃) aufweist.

3. Einrichtung (20) nach einem der Ansprüche 1-2, wobei:
(a) das Gehäuse (24) derart geformt ist, dass es eine Füllöffnung (34) definiert, die in Fluidverbindung mit dem Reservoir (22) steht, und wobei das Reservoir (22) dazu konfiguriert ist, über die Füllöffnung (34) mit der therapeutischen Substanz gefüllt zu werden,
(b) vor dem Befüllen des Reservoirs (22) sich die Einrichtung (20) in einem Ruhezustand befindet, der Kolben (26) in einer anfänglichen proximalen Position innerhalb des Reservoirs (22) angeordnet ist, und sich der Positionssensor (30) in dem Zustand S₁ befindet,
(c) das Füllen des Reservoirs (22) mit der therapeutischen Substanz bewirkt, dass der Kolben (26) von der anfänglichen proximalen Position in eine distale Richtung gleitet,
(d) der Kolben (26) und der Positionssensor (30) derart angeordnet sind, dass, wenn der Kolben (26) in der distalen Richtung gleitet, die Kolben-Sensor-Eingriffsschnittstelle (28) veranlasst, dass der Positionssensor (30) den Zustand von S₁ zu S₂ ändert, und
(e) die Steuerschaltung (32) dazu konfiguriert ist, zu identifizieren, dass die Einrichtung (20) wach ist und sich in einem Füllzustand befindet, als Reaktion darauf, dass der Positionssensor (30) den Zustand von S₁ nach S₂ ändert.

4. Einrichtung (20) nach Anspruch 3, wobei:
der Kolben (26) und der Positionssensor (30) derart angeordnet sind, dass nach Beendigung des Füllens des Reservoirs (22) mit einem Schwellenvolumen der therapeutischen Substanz die Kolben-Sensor-Eingriffsschnittstelle (28) den Positionssensor (30) veranlasst, den Zustand von S₂ zurück nach S₁ zu ändern, und
die Steuerschaltung (32) dazu konfiguriert ist, zu identifizieren, dass ein Füllfehler aufgetreten ist, als Reaktion darauf, dass (i) der Zustand der Einrichtung (20) füllend ist, in Kombination mit (ii) einer Füllschwellenzeitspanne, die ab dem Zeitpunkt verstrichen ist, zu dem der Positionssensor (30) den Zustand von S₁ nach S₂ wechselt, ohne dass der Positionssensor (30) den Zustand von S₂ zurück nach S₁ ändert.

5. Einrichtung (20) nach Anspruch 3, wobei der Kolben (26) und der Positionssensor (30) derart angeordnet sind, dass die Kolben-Sensor-Eingriffsschnittstelle (28) nach Beendigung des Füllens des Reservoirs (22) mit wenigstens einem Schwellenvolumen der therapeutischen Substanz veranlasst, dass der Positionssensor (30) den Zustand von S₂ zurück zu S₁ ändert, und wobei die Steuerschaltung (32) dazu konfiguriert ist, zu identifizieren, dass sich die Einrichtung (20) in einem Zustand der vollständigen Füllung befindet, als Reaktion darauf, dass der Positionssensor (30) den Zustand von S₂ zurück zu S₁ ändert.

6. Einrichtung (20) nach Anspruch 5, wobei:
der Kolben (26) und der Positionssensor (30) derart angeordnet sind, dass, nachdem die Kolben-Sensor-Eingriffsschnittstelle (28) veranlasst hat, dass der Positionssensor (30) den Zustand von S₁ nach S₂ ändert, die Kolben-Sensor-Eingriffsschnittstelle (28) den Eingriff mit dem Positionssensor (30) aufrechterhält, um den Positionssensor (30) in dem Zustand S₂ zu halten, bis das Reservoir (22) mit dem Schwellenvolumen der therapeutischen Substanz gefüllt worden ist, und
der Kolben (26) und der Positionssensor (30) derart angeordnet sind, dass eine weitere distale Bewegung des Kolbens (26), nachdem das Reservoir (22) mit dem Schwellenvolumen der therapeutischen Substanz gefüllt wurde, veranlasst, dass die Kolben-Sensor-Eingriffsschnittstelle (28) den Eingriff mit dem Positionssensor (30) beendet, was veranlasst, dass der Positionssensor (30) von S₂ zurück zu S₁ schaltet.

7. Einrichtung (20) nach Anspruch 3, wobei:
(a) nach Beendigung der Befüllung des Reservoirs (22) mit wenigstens einem Schwellenvolumen der therapeutischen Substanz die Einrichtung (20) sich in einem Zustand der vollständigen Befüllung befindet und der Positionssensor (30) sich in dem Zustand S₁ befindet,
(b) die Steuerschaltung (32), nachdem sich die Einrichtung (20) in dem Zustand der vollständigen Befüllung befindet, dazu konfiguriert ist, einen Zustand der begonnenen Abgabe der Einrichtung (20) zu initiieren, in dem die Steuerschaltung (32) die Abgabeeinrichtung (20) für eine therapeutische Substanz antreibt, um die therapeutische Substanz von dem Reservoir (22) an ein Subjekt abzugeben, indem der Kolben (26) angetrieben wird, um innerhalb des Reservoirs (22) proximal zu gleiten, und
(c) der Kolben (26) und der Positionssensor (30) derart angeordnet sind, dass, wenn der Kolben (26) proximal gleitet, die Kolben-Sensor-Eingriffsschnittstelle (28) veranlasst, dass der Positionssensor (30) den Zustand von S₁ zu S₃ ändert.

8. Einrichtung (20) nach Anspruch 7, wobei die Steuerschaltung (32) ferner dazu konfiguriert ist, (a) eine Eingabe zu empfangen, die ein beabsichtigtes Volumen der therapeutischen Substanz angibt, das in das Reservoir (22) gefüllt werden soll, und (b) als Reaktion auf die empfangene Eingabe die Länge einer erwarteten Wartezeit zwischen dem Zeitpunkt, an dem die Steuerschaltung (32) den Zustand der begonnenen Abgabe der Einrichtung (20) initiiert, und dem Zeitpunkt zu bestimmen, an dem die Kolben-Sensor-Eingriffsschnittstelle (28) veranlasst, dass der Positionssensor (30) den Zustand von S₁ zu S₃ ändert.

9. Einrichtung (20) nach Anspruch 8, wobei:
(a) die Steuerschaltung (32) dazu konfiguriert ist, zu identifizieren, dass das Reservoir (22) mit einem Volumen an therapeutischer Substanz gefüllt wurde, das größer als das beabsichtigte Volumen ist, als Reaktion darauf, dass (i) der aktuelle Zustand der Einrichtung (20) der Zustand der begonnenen Abgabe ist, in Kombination damit (ii), dass der Positionssensor (30) nach einer Zeitspanne, die länger als die erwartete Wartezeit, aber kürzer als eine Schwellenzeitspanne des Abgabebeginns ist, den Zustand von S₁ zu S₃ ändert,
(b) die Steuerschaltung (32) dazu konfiguriert ist, die Volumendifferenz zwischen dem beabsichtigten Volumen und dem Volumen der therapeutischen Substanz innerhalb des Reservoirs (22), das größer als das beabsichtigte Volumen ist, zu kompensieren, indem die Abgabe der therapeutischen Substanz an das Subjekt nach Abgabe des beabsichtigten Volumens an das Subjekt beendet wird,
(c) der Kolben (26) und der Positionssensor (30) derart angeordnet sind, dass als Reaktion darauf, dass das Reservoir (22) mit dem beabsichtigten Volumen gefüllt wird, wenn das beabsichtigte Volumen an das Subjekt abgegeben wird, die Kolben-Sensor-Eingriffsschnittstelle (28) veranlasst, dass der Positionssensor (30) den Zustand von S₃ zurück zu S₁ zu ändert, und
(d) als Reaktion auf die Identifizierung, dass das Reservoir (22) mit einem Volumen an therapeutischer Substanz gefüllt wurde, das größer ist als das beabsichtigte Volumen, wenn das beabsichtigte Volumen an das Subjekt abgegeben wird, die Steuerschaltung (32) dazu konfiguriert ist, die Abgabe der therapeutischen Substanz an das Subjekt zu beenden, obwohl der Positionssensor (30) in dem Zustand S₃ verbleibt.

10. Einrichtung (20) nach Anspruch 8, wobei die Steuerschaltung (32) dazu konfiguriert ist, zu identifizieren, dass sich die Einrichtung (20) in einem Zustand der laufenden Abgabe befindet, in dem therapeutische Substanz aus dem Reservoir (22) an das Subjekt abgegeben wird, als Reaktion darauf, dass der Positionssensor (30) den Zustand von S₁ zu S₃ ändert, nachdem die Steuerschaltung (32) den Zustand der begonnenen Abgabe der Einrichtung (20) initiiert hat und die erwartete Wartezeit verstrichen ist.

11. Einrichtung (20) nach Anspruch 10, wobei der Kolben (26) und der Positionssensor (30) derart angeordnet sind, dass nach Beendigung der Abgabe des beabsichtigten Volumens der therapeutischen Substanz aus dem Reservoir (22) die Kolben-Sensor-Eingriffsschnittstelle (28) veranlasst, dass der Positionssensor (30) den Zustand von S₃ zurück zu S₁ ändert, und wobei die Steuerschaltung (32) dazu konfiguriert ist, zu identifizieren, dass sich die Einrichtung (20) in einem Zustand der vollständigen Abgabe befindet, als Reaktion darauf, dass der Positionssensor (30) den Zustand von S₃ zu S₁ ändert.

12. Einrichtung (20) nach Anspruch 11, wobei die Steuerschaltung (32) dazu konfiguriert ist, zu identifizieren, dass ein Fehler der laufenden Abgabe aufgetreten ist, als Reaktion darauf, dass (i) der aktuelle Zustand der Einrichtung (20) der Zustand der laufenden Abgabe ist, in Kombination damit (ii), dass eine Schwellenzeitspanne der laufenden Abgabe ab dem Zeitpunkt verstrichen ist, zu dem der Positionssensor (30) den Zustand von S₁ zu S₃ ändert, ohne dass der Positionssensor (30) den Zustand von S₃ zurück zu S₁ ändert.

13. Einrichtung (20) nach einem der Ansprüche 1-2, wobei die Einrichtung (20) dazu konfiguriert ist, dass, wenn sich das Reservoir (22) in einem gefüllten Zustand befindet, in dem ein Volumen einer therapeutischen Substanz innerhalb des Reservoirs (22) angeordnet ist, und sich der Positionssensor (30) in dem Zustand S₁ befindet:
(a) die Steuerschaltung (32) dazu konfiguriert ist, einen Zustand der begonnenen Abgabe der Einrichtung (20) zu initiieren, in dem die Steuerschaltung (32) die Abgabeeinrichtung (20) für eine therapeutische Substanz antreibt, um die therapeutische Substanz aus dem Reservoir (22) an ein Subjekt abzugeben, indem der Kolben (26) angetrieben wird, proximal in dem Reservoir (22) zu gleiten, und
(b) der Kolben (26) und der Positionssensor (30) derart angeordnet sind, dass, wenn der Kolben (26) proximal gleitet, die Kolben-Sensor-Eingriffsschnittstelle (28) veranlasst, dass der Positionssensor (30) den Zustand von S₁ in einen Zustand nach dem ersten ändert.

## Revendications

1. Appareil d'administration de substance thérapeutique (20) comprenant :
un boîtier (24) ;
un réservoir (22) disposé au moins partiellement à l'intérieur du boîtier (24) et conçu pour contenir une substance thérapeutique ;
un capteur de position (30) ayant trois états distincts (S₁, S₂, S₃) ;
un piston (26) susceptible de coulisser à l'intérieur du réservoir (22) ;
une interface (28) de contact piston-capteur couplée au piston (26) à l'intérieur du réservoir (22) et s'étendant de l'intérieur du réservoir (22) vers l'extérieur du réservoir (22), ledit piston (26) et ledit capteur de position (30) étant agencés de telle façon que l'interface (28) de contact piston-capteur amène le capteur de position (30) à changer d'état lorsque le piston (26) coulisse à l'intérieur du réservoir (22) ; et
un circuit de commande (32) conçu pour identifier une nouvelle indication d'état de l'appareil (20) en fonction (a) d'un changement d'état du capteur de position (30), ou (b) d'un état précédent ou actuel de l'appareil (20) en association avec un seuil de temps écoulé sans que le capteur de position (30) n'ait changé d'état,
dans lequel :
le piston (26) et le capteur de position (30) sont agencés de telle façon qu'un mouvement distal du piston (26), d'une position proximale initiale à l'intérieur du réservoir (22) vers une position intermédiaire à l'intérieur du réservoir (22), amène l'interface (28) de contact piston-capteur à changer l'état du capteur de position (30) d'un premier état S₁ parmi lesdits états distincts, dans lequel l'interface (28) de contact piston-capteur n'est pas en contact avec le capteur de position (30), vers un deuxième état S₂ parmi lesdits états distincts, dans lequel l'interface (28) de contact piston-capteur est en contact avec le capteur de position (30) à mesure que le piston (26) subit un coulissement distal à l'intérieur du réservoir (22),
le piston (26) et le capteur de position (30) sont agencés de telle façon qu'un mouvement distal du piston (26) au-delà de la position intermédiaire vers une position post-intermédiaire à l'intérieur du réservoir (22) amène l'interface (28) de contact piston-capteur à changer l'état du capteur de position (30), qui passe de S₂ pour revenir à S₁,
le piston (26) et le capteur de position (30) sont agencés de telle façon que, à la suite du changement d'état du capteur de position (30), qui passe de S₂ pour revenir à S₁, un mouvement proximal du piston (26) à l'intérieur du réservoir (22) amène l'interface (28) de contact piston-capteur à changer l'état du capteur de position (30) de S₁ vers un troisième état S₃ parmi lesdits états distincts, dans lequel l'interface (28) de contact piston-capteur est en contact avec le capteur de position (30) à mesure que le piston (26) subit un coulissement proximal à l'intérieur du réservoir (22), et
le piston (26) et le capteur de position (30) sont agencés de telle façon que, à la suite du passage du capteur de position (30) de S₁ à S₃, un prolongement de mouvement proximal du piston (26) amène l'interface (28) de contact piston-capteur à changer l'état du capteur de position (30), qui passe de S₃ pour revenir à S₁.

2. Appareil (20) selon la revendication 1, dans lequel le capteur de position (30) a exactement trois états distincts (S₁, S₂, S₃).

3. Appareil (20) selon l'une quelconque des revendications 1 et 2, dans lequel :
(a) le boîtier (24) est formé pour définir un orifice de remplissage (34) en communication fluidique avec le réservoir (22), ledit réservoir (22) étant conçu pour être rempli de substance thérapeutique par l'intermédiaire de l'orifice de remplissage (34),
(b) avant le remplissage du réservoir (22), l'appareil (20) est en état de veille, le piston (26) est disposé dans une position initiale proximale à l'intérieur du réservoir (22), et le capteur de position (30) est à l'état S₁,
(c) le remplissage du réservoir (22) avec la substance thérapeutique amène le piston (26) à coulisser dans une direction distale à partir de la position proximale initiale,
(d) le piston (26) et le capteur de position (30) sont agencés de telle façon que, à mesure que le piston (26) coulisse dans la direction distale, l'interface (28) de contact piston-capteur amène le capteur de position (30) à changer d'état de S₁ à S₂, et
(e) le circuit de commande (32) est conçu pour identifier que l'appareil (20) est éveillé et en état de remplissage en réponse au changement d'état du capteur de position (30) de S₁ à S₂.

4. Appareil (20) selon la revendication 3, dans lequel :
le piston (26) et le capteur de position (30) sont agencés de telle façon que, une fois le réservoir (22) rempli d'un volume seuil de substance thérapeutique, l'interface (28) de contact piston-capteur amène le capteur de position (30) à changer d'état, passant de S₂ pour revenir à S₁, et
le circuit de commande (32) est conçu pour identifier qu'une erreur de remplissage s'est produite en réponse au fait que (i) l'état de l'appareil (20) est un état de remplissage, en association avec le fait que (ii) une durée seuil de remplissage s'est écoulée à partir du moment où le capteur de position (30) a changé d'état, passant de S₁ pour revenir à S₂, sans que le capteur de position (30) n'ait changé d'état en passant de S₂ pour revenir à S₁.

5. Appareil (20) selon la revendication 3, dans lequel le piston (26) et le capteur de position (30) sont agencés de telle façon que, une fois le réservoir (22) rempli d'au moins un volume seuil de substance thérapeutique, l'interface (28) de contact piston-capteur amène le capteur de position (30) à changer d'état, passant de S₂ pour revenir à S₁, et dans lequel le circuit de commande (32) est conçu pour identifier que l'appareil (20) est dans un état de remplissage terminé en réponse au changement d'état du capteur de position (30) qui passe de S₂ pour revenir à S₁.

6. Appareil (20) selon la revendication 5, dans lequel :
le piston (26) et le capteur de position (30) sont agencés de telle façon que, à la suite du fait que l'interface (28) de contact piston-capteur a amené le capteur de position (30) à changer d'état, passant de S₁ à S₂, l'interface (28) de contact piston-capteur maintient le contact avec le capteur de position (30), de manière à maintenir le capteur de position (30) à l'état S₂, jusqu'à ce que le réservoir (22) ait été rempli dudit volume seuil de substance thérapeutique, et
le piston (26) et le capteur de position (30) sont agencés de telle façon qu'un prolongement de mouvement distal du piston (26) à la suite du remplissage du réservoir (22) avec ledit volume seuil de substance thérapeutique amène l'interface (28) de contact piston-capteur à mettre fin au contact avec le capteur de position (30), amenant le capteur de position (30) à passer de S₂ pour revenir à S₁.

7. Appareil (20) selon la revendication 3, dans lequel :
(a) une fois que le réservoir (22) a fini de se remplir d'au moins un volume seuil de substance thérapeutique, l'appareil (20) est dans un état de remplissage terminé, et le capteur de position (30) est à l'état S₁,
(b) à la suite du fait que l'appareil (20) est à l'état de remplissage terminé, le circuit de commande (32) est conçu pour amorcer un état de commencement d'administration de l'appareil (20), dans lequel le circuit de commande (32) amène l'appareil d'administration de substance thérapeutique (20) à administrer la substance thérapeutique du réservoir (22) à un sujet, en entraînant le coulissement proximal du piston (26) à l'intérieur du réservoir (22), et
(c) le piston (26) et le capteur de position (30) sont agencés de telle façon que, à mesure que le piston (26) subit le coulissement proximal, l'interface (28) de contact piston-capteur amène le capteur de position (30) à changer d'état, passant de S₁ à S₃.

8. Appareil (20) selon la revendication 7, dans lequel le circuit de commande (32) est en outre conçu pour (a) recevoir une entrée indiquant un volume prévu de substance thérapeutique à remplir dans le réservoir (22), et (b) en réponse à l'entrée reçue, déterminer la durée d'un temps d'attente prévu entre le moment où le circuit de commande (32) amorce l'état de commencement d'administration de l'appareil (20) et le moment où l'interface (28) de contact piston-capteur amène le capteur de position (30) à passer de l'état S₁ à S₃.

9. Appareil (20) selon la revendication 8, dans lequel :
(a) le circuit de commande (32) est conçu pour identifier que le réservoir (22) a été rempli d'un volume de substance thérapeutique supérieur au volume prévu en réponse au fait que (i) l'état actuel de l'appareil (20) est un état de commencement d'administration, en association avec le fait que (ii) le capteur de position (30) a changé d'état, passant de S₁ à S₃ après une durée supérieure à la durée d'attente prévue mais inférieure à une durée seuil de commencement d'administration,
(b) le circuit de commande (32) est conçu pour compenser la différence de volume entre le volume prévu et un volume de substance thérapeutique à l'intérieur du réservoir (22) qui est supérieur au volume prévu, en mettant fin à l'administration de la substance thérapeutique au sujet une fois que le volume prévu a été administré au sujet,
(c) le piston (26) et le capteur de position (30) sont agencés de telle façon que, en réponse au fait que le réservoir (22) est rempli dudit volume prévu et une fois que le volume prévu a été administré au sujet, l'interface (28) de contact piston-capteur amène le capteur de position (30) à changer d'état, passant de S₃ pour revenir à S₁, et
(d) en réponse à l'identification que le réservoir (22) a été rempli d'un volume de substance thérapeutique qui est supérieur au volume prévu, une fois que le volume prévu a été administré au sujet, le circuit de commande (32) est conçu pour mettre fin à l'administration de la substance thérapeutique au sujet malgré le fait que le capteur de position (30) demeure à l'état S₃.

10. Appareil (20) selon la revendication 8, dans lequel le circuit de commande (32) est conçu pour identifier que l'appareil (20) est dans un état d'administration en cours, dans lequel la substance thérapeutique est administrée du réservoir (22) au sujet, en réponse au changement d'état du capteur de position (30), qui passe de S₁ à S₃ à la suite de l'amorce, par le circuit de commande (32), de l'état de commencement d'administration de l'appareil (20) et à l'écoulement de la durée d'attente prévue.

11. Appareil (20) selon la revendication 10, dans lequel le piston (26) et le capteur de position (30) sont agencés de telle façon que, une fois que l'administration de substance thérapeutique issue du réservoir (22) selon le volume prévu est terminée, l'interface (28) de contact piston-capteur amène le capteur de position (30) à changer d'état, passant de S₃ pour revenir à S₁, et dans lequel le circuit de commande (32) est conçu pour identifier que l'appareil (20) est dans un état d'administration terminée en réponse au changement d'état du capteur de position (30) de S₃ à S₁.

12. Appareil (20) selon la revendication 11, dans lequel le circuit de commande (32) est conçu pour identifier qu'une erreur de progression d'administration s'est produite en réponse au fait que (i) l'état actuel de l'appareil (20) est celui d'une administration en cours, en association avec le fait que (ii) une durée seuil de progression d'administration s'est écoulée à partir du moment où le capteur de position (30) a changé d'état pour passer de S₁ à S₃ sans que le capteur de position (30) n'ait changé d'état en passant de S₃ pour revenir à S₁.

13. Appareil (20) selon l'une quelconque des revendications 1 et 2, dans lequel l'appareil (20) est conçu de telle façon que, lorsque le réservoir (22) est dans un état rempli dans lequel un volume de substance thérapeutique est présent dans le réservoir (22), et que le capteur de position (30) est à l'état S₁ :
(a) le circuit de commande (32) est conçu pour amorcer un état de commencement d'administration de l'appareil (20) dans lequel le circuit de commande (32) amène l'appareil d'administration de substance thérapeutique (20) à administrer la substance thérapeutique du réservoir (22) à un sujet en entraînant le coulissement proximal du piston (26) à l'intérieur du réservoir (22), et
(b) le piston (26) et le capteur de position (30) sont agencés de telle façon que, à mesure que le piston (26) subit le coulissement proximal, l'interface (28) de contact piston-capteur amène le capteur de position (30) à changer d'état, passant de S₁ à un post-premier état.
